(19) Europäisches Patentamt
European Patent Office
Office européen des brevets

(11) **EP 1 875 932 A1**

(12) **EUROPEAN PATENT APPLICATION**
published in accordance with Art. 153(4) EPC

(43) Date of publication:
**09.01.2008 Bulletin 2008/02**

(51) Int Cl.:
*A61M 1/36* (2006.01)    *B01J 20/26* (2006.01)

(21) Application number: **06730474.1**

(22) Date of filing: **29.03.2006**

(86) International application number:
**PCT/JP2006/306526**

(87) International publication number:
**WO 2006/106763 (12.10.2006 Gazette 2006/41)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **30.03.2005 JP 2005099525**

(71) Applicant: **Kaneka Corporation
Kita-ku
Osaka-shi, Osaka 530-8288 (JP)**

(72) Inventors:
• **KOBAYASHI, Akira,
KANEKA CORPORATION
Takasago-shi,
Hyogo 676-8688 (JP)**

• **YOSHIDA, Shinya,
KANEKA CORPORATION
Takasago-shi,
Hyogo 676-8688 (JP)**
• **NIWA, Hideo,
KANEKA CORPORATION
Takasago-shi,
Hyogo 676-8688 (JP)**

(74) Representative: **Gille Hrabal Struck Neidlein Prop
Roos
Patentanwälte
Brucknerstrasse 20
40593 Düsseldorf (DE)**

(54) **METHOD OF REMOVING LYMPHOCYTE GROWTH INHIBITOR**

(57)    The present invention relates to a method of body fluid treatment for relieving the condition of inhibited lymphocyte proliferation which comprises bringing a mammalian body fluid into contact with an adsorbent comprising a high-molecular compound having a water contact angle within the range of 40° to 98° in the presence of a divalent cation chelating agent in the manner of extracorporeal circulation, or comprises bringing a mammalian body fluid into contact with an active carbon-containing adsorbent in the manner of extracorporeal circulation; and relates to a system for extracorporeal circulation which comprises a body fluid transfer pump, an anticoagulant infusion pump, and a device comprising a container wherein an adsorbent comprising a high-molecular compound having a water contact angle within the range of 40° to 98° is contained, or comprises a body fluid transfer pump, and a device comprising a container wherein an active carbon-containing adsorbent is contained.

**Description**

TECHNICAL FIELD

The present invention relates to a system for extracorporeal circulation and a method of body fluid treatment for relieving a patient with a cancer, infectious disease, immunologic disease or like disease in which immunocompetent cells (in particular lymphocytes) in the blood are in a condition of poor proliferation or inhibited proliferation from the condition of inhibited lymphocyte proliferation by adsorptively removing a lymphocyte proliferation inhibiting factor or factors from the blood through extracorporeal circulation.

BACKGROUND ART

In recent years, attention has been focused on activated autologous lymphocyte therapy, which comprises taking immunocompetent cells (lymphocytes in particular) in blood out of the body, culturing them for stimulation/activation and for proliferation and again returning them into the body to thereby prevent the advance of cancer. This technique produces little side effects and makes it possible to maintain the quality of life (QOL) at high levels even during treatment and, therefore, is becoming more and more popular in the field of cancer treatment as a fourth choice of cancer therapy next to the three major cancer treatment methods, namely surgical therapy, radiotherapy and chemotherapy. The technique is already in actual use as one of tailor-made highly advanced medical treatment methods in university hospitals, cancer centers and specialized clinics and, expectedly, it will be used still more widely. This technique generally comprises collecting a portion of the blood of a patient, separating a lymphocyte fraction by density gradient centrifugation, adding the autologous plasma to a medium for exclusive use and cultivating the lymphocytes. Generally, the number of lymphocytes arrives at about 100 times the number of lymphocytes in the primary culture in a week. It has become known, however, that there are some such cancer patients that lymphocytes derived therefrom can hardly proliferate in the presence of autologous plasma but can proliferate in the presence of the plasma derived from another person (resulting from blood donation). This suggests the presence of a factor, in the autologous plasma, which inhibits the lymphocyte proliferation. Cancer cells produce cellular immunity inhibiting factors, for example such cytokines as transforming growth factor beta (hereinafter abbreviated as TGF-β), interleukin 4 (hereinafter abbreviated as IL4), interleukin 6 (hereinafter abbreviated as IL6) and interleukin 10 (hereinafter abbreviated as IL10) as well as prostaglandin E2 (hereinafter abbreviated as PGE2). Thus, the possibility is suggested that such factors might inhibit the lymphocyte proliferation. However, the concentrations of these factors in the blood of cancer patients are almost the same as those in persons in normal health, although their local concentrations in cancer foci are high. Further, when such factors commercially available as reagents are dissolved in plasma at high concentrations and the influences thereof on lymphocyte proliferation are examined, little inhibition is observed. Such and other findings suggest that there is some lymphocyte proliferation inhibiting factor other than each of such factors and cytokines which strongly inhibit lymphocyte proliferation.
As a matter of fact, an adsorbent for adsorptively removing immunosuppressive acidic proteins (IAPs) (Patent Document 1), an adsorbent for adsorptively removing interleukins in body fluids (Patent Document 2) and an adsorbent capable of adsorbing TGF-β in body fluids (Patent Document 3), among others, have so far been disclosed. However, all of them are limited in scope to the adsorptive removal of cytokines and there is no report about an adsorbent capable of improving the proliferative activity of lymphocytes. In recent years, the number of patients having activated lymphocyte therapy has been increasing year by year with the marked advance of such therapy and, on the other hand, the number of patients relying on blood donation because of poor lymphocyte proliferation has also been increasing. In the case of blood donation, it is necessary to secure non-autologous plasma in conformity with patient's therapeutic cycle. Further, there are a number of problems to be taken up from the safety viewpoint, for example the risk of infection; therefore, it is desired that a method for relieving the lymphocyte proliferation inhibition in lymphocyte culture by a simple procedure without losing other useful substances be developed. Furthermore, in cancer patients as well whose lymphocyte can proliferate in the presence of autologous body fluids, it is expected that further improvements in proliferation rate and in cytokine producing activity, for instance, be achieved when the inhibition of lymphocyte proliferation in lymphocyte culture is broken down. Currently, however, neither adsorbent, nor porous material, nor device nor treatment method is available for such purposes. Further, adsorbents prepared by causing a material capable of adsorbing cytokines and like immunosuppressive proteins to bind to a water-insoluble carrier have been disclosed (Patent Document 4 to 6). However, the effect of those adsorbents depends on an affinity between an amine residue and such immunosuppressive proteins as cytokines. Therefore, those adsorbents require the presence of an amine residue.
At present, a treatment is eagerly anticipated which will promote the proliferation of those lymphocytes which are in a poorly proliferative condition for the treatment of a cancer, infectious disease, immunologic disease or like disease in which immunocompetent cells (in particular lymphocytes) in the blood are in a condition of poor proliferation or inhibited proliferation (hereinafter, this treatment is also called as "a relieve of inhibited lymphocyte proliferation").
Accordingly, it is an object of the invention to provide a method of body fluid treatment and a system for extracorporeal circulation which can relieve the condition of inhibited lymphocyte proliferation and thereby improve the lymphocyte proliferation of and allows the passage of a body fluid without loss of useful substances in the body fluid.
Patent Document 1: Japanese Kokai Publication Sho-56-092824
Patent Document 2: Japanese Kokai Publication Hei-08-257398
Patent Document 3: Japanese Kokai Publication 2001-218840
Patent Document 4: Japanese Kokai Publication 2003-310751
Patent Document 5: Japanese Kokai Publication 2003-339854
Patent Document 6: Japanese Kokai Publication 2004-73618

SUMMARY OF THE INVENTION

The present inventors made intensive investigations and, as a result, found that when a body fluid is brought into contact with an adsorbent comprising a high-molecular compound having a water contact angle within the range of 40°C to 98°C in the presence of a divalent cation chelating agent or a body fluid is brought into contact with an active carbon-containing adsorbent, the condition of inhibited lymphocyte proliferation can be relieved and the lymphocyte proliferation can be improved without substantial loss of blood cells, in the body fluid, such as erythrocytes, leukocytes and platelets, while maintaining good blood passage. Such findings have now led to completion of the present invention.

Thus, the present invention relates to
a method of body fluid treatment for relieving the condition of inhibited lymphocyte proliferation
which comprises bringing a mammalian body fluid into contact with an adsorbent comprising a high-molecular compound having a water contact angle within the range of 40° to 98° in the presence of a divalent cation chelating agent in the manner of extracorporeal circulation;
a method of body fluid treatment for relieving the condition of inhibited lymphocyte proliferation
which comprises bringing a mammalian body fluid into contact with an active carbon-containing adsorbent in the manner of extracorporeal circulation;
a system for extracorporeal circulation
which comprises a body fluid transfer pump, an anticoagulant infusion pump, and a device comprising a container wherein an adsorbent comprising a high-molecular compound having a water contact angle within the range of 40° to 98° is contained; and
a system for extracorporeal circulation
which comprises a body fluid transfer pump, and a device comprising a container wherein an active carbon-containing adsorbent is contained.

DETAILED DESCRIPTION OF THE INVENTION

In the following, the present invention is described in detail.
First, the adsorbent of the invention is described.
The adsorbent of the invention is an adsorbent which comprises a high-molecular compound having a water contact angle within the range of 40° to 98°. The adsorbent of the invention may be used in extracorporeal circulation of a body fluid.
In the practice of the invention, the water contact angle can be determined by preparing a smooth film specimen constituted of the high-molecular compound, which is the main constituent of the adsorbent, forming a liquid drop on the film in a horizontal state using a microinjector and measuring the contact angle at room temperature. When the high-molecular compound is soluble in an organic solvent, the contact angle can also be measured after dissolving the high-molecular compound and preparing a cast film specimen on a flat sheet using the resulting solution. For details of the measurement method, reference can be made, for example, to "Shin-Jikken Kagaku Koza (Lectures in Experimental Chemistry, New Series) 18: Kaimen to Koroido (Interface and Colloid)" (First Edition, published October 20, Showa 52 (1977) by Maruzen Co., Ltd.).
Thus, a flat sheet specimen having a mirror-finish level of smoothness is placed horizontally so that the atmosphere surrounding the same may be filled with the saturated vapor of the liquid to be subjected to measurement, and a liquid drop is formed thereon using a microinjector. The size of the liquid drop is such that the contact diameter is about 3 mm or smaller. The contact angle can be determined by measuring the angle formed upon allowing the liquid drop to advance toward the solid surface (at the time when after the liquid is allowed to develop and spread on the specimen, the liquid drop becomes stable at a certain size) using a reading microscope (having a magnification of about 20) equipped with a goniometer. The visibility of the image becomes very good when the lens barrel is inclined by 1 to 2 degrees downward from the horizontal. The drop is illuminated from the front with light transmitted through an opalescent glass or with parallel beams of light transmitted through a heat ray-absorbing glass.
The contact angle data reported herein were measured by the method described later in the example section.

The high-molecular compound having a water contact angle within the range of 40° to 98° includes as typical examples, but is not limited to, synthetic high-molecular compounds such as nylon 6, nylon 6, 6, nylon 11, polyethylene, poly (vinylidene chloride), poly(vinyl chloride), poly(vinyl acetate), polystyrene, styrene-divinylbenzene copolymers, poly (trifluoroethylene), poly(chlorotrifluoroethylene), poly(ethylene terephthalate), polypropylene, polyacrylic esters (e.g. poly (methyl acrylate)), polymethacrylic esters (e.g. poly(methyl methacrylate)), crosslinked polyacrylates and crosslinked polyamides as well as cellulose and like water-insoluble ones. Among them, polymers or copolymers produced by polymerizing an aromatic monomer or monomers (e. g. monomers selected from among styrene; alkylstyrenes which may optionally be substituted, for example methylstyrene and ethylstyrene; divinylbenzene; and benzo-condensed cyclic compounds which may optionally be substituted, for example divinylnaphthalene and divinylanthracene) are preferred from the lymphocyte proliferation rate viewpoint. In particular, polystyrene and styrene-divinylbenzene copolymers are preferred.

Any polystyrene species can be used as the polystyrene. The polystyrene is a homopolymer of styrenes (styrene; alkylstyrenes which may optionally be substituted, for example methylstyrene or ethylstyrene) or a copolymer of those styrenes.

The styrene-divinylbenzene copolymers can be obtained by crosslinking the above-mentioned styrenes with m-, o- or p-divinylbenzene, which may optionally be substituted.

The high-molecular compound mentioned above may optionally be substituted by a halogen, alkyl, alkenyl, alkynyl, aralkyl, aryl, heteroaryl, arylcarbonyl, heteroarylcarbonyl, alkylcarbonyl, alkoxycarbonyl, arylsulfonyl, heteroarylsulfonyl, alkylsulfonyl, alkylaminosulfonyl, etc. The substituents mentioned above may further be substituted by a substituent(s) other than amines, and such substituents on the above-mentioned substituents are preferably other than amide, urea, ester and ether groups.

The high-molecular compound mentioned above is preferably one having no amine group bound thereto. As the amine group, there may be mentioned those groups resulting from chemical bonding of ammonia, primary to tertiary amine or the like to the high-molecular compound.

Further, the high-molecular compound mentioned above is preferably one comprising no other compound immobilized thereon. The other compound is not particularly restricted provided that they are not the high-molecular compound mentioned above but includes, for example, amines, alcohols, glycidyl ethers, carboxylic acids and derivatives thereof, acid halides, halides, halogenated silanes, thiols, aldehydes and antibodies.

For the purpose of the present invention, namely from the lymphocyte proliferation promotion viewpoint, the above-mentioned water contact angle is preferably about 60° to 96°, more preferably about 70° to 94°, still more preferably about 75° to 91°. When the water contact angle is less than 40°, the hydrophilicity of the adsorbent is high, so that, presumably, the adsorption of proteins and other substances involved in the inhibition of lymphocyte proliferation decreases and it becomes difficult to relieve the condition of inhibited lymphocyte proliferation. When, on the other hand, the water contact angle is greater than 98°, the hydrophobicity of the adsorbent becomes still higher and the capacity to adsorb proteins and so forth tends to decrease. As a result, presumably, the adsorption of proteins and other substances involved in the inhibition of lymphocyte proliferation also decreases and it becomes difficult to relieve the condition of inhibited lymphocyte proliferation.

In the practice of the invention, the adsorbent is preferably insoluble in water.

The adsorbent of the invention contains not less than about 50% by weight, preferably not less than about 60% by weight, more preferably not less than about 70% by weight, still more preferably not less than 80% by weight, of the high-molecular compound mentioned above.

As the component which can be contained in the adsorbent in addition to the above-mentioned high-molecular compound having a water contact angle within the range of 40° to 98°, there may be mentioned, for example, polyvinyl alcohol (contact angle 36°), poly(hydroxyethyl methacrylate) (contact angle 13°) and paraffin (contact angle 105 to 106°), among others. By using two or more of the high-molecular compounds mentioned above having a water contact angle within the range of 40° to 98° in combination and/or by using any of the above-mentioned high-molecular compounds having a water contact angle of 40° to 98 ° as the main component and incorporating a component other than the high-molecular compound as the auxiliary material, among others, it is possible to adjust the water contact angle of the high-molecular compound to be obtained.

The adsorbent of the invention occurs as a solid at ordinary temperature and ordinary pressure and has pores appropriate in size, namely is preferably a porous material having a porous structure.

As for the size of pores in the porous material, the molecular-weight exclusion limit of the porous material as determined using polystyrene beads is preferably not higher than $1.5 \times 10^5$, more preferably not higher than $1.4 \times 10^5$. When the molecular-weight exclusion limit is higher than $1.5 \times 10^5$, the level of unspecific adsorption tends to increase, the loss of useful proteins in the body fluid tends to occur and/or the lymphocyte proliferation tends to decrease with ease. For lessening the influence of unspecific adsorption while maintaining the lymphocyte proliferation rate at high levels, the molecular-weight exclusion limit is more preferably not higher than $1.3 \times 10^5$, particularly preferably not higher than $1.2 \times 10^5$, most preferably not higher than $1.0 \times 10^5$.

The molecular-weight exclusion limit can be easily controlled, for example, by adjusting the content of the above-mentioned high-molecular compound, which is the main constituent of the porous material, in the step of porous material production. Thus, as the content of the high-molecular compound increases, the molecular-weight exclusion limit lowers and, as the content of the high-molecular compound decreases, the molecular-weight exclusion limit rises.

The molecular-weight exclusion limit can be measured in the following manner. Polystyrene beads differing in particle diameter are passed through a column packed with the adsorbent, and the pore size is determined on the basis of an excluded polystyrene beads-based exclusion curve. Then, the above-mentioned pore size is extrapolated to a spherical protein (e.g. dextran) the diameter and molecular weight of which are known, to thereby determine the molecular weight on the spherical protein equivalent basis; this is regarded as the molecular-weight exclusion limit.

As for the shape or form of the adsorbent, any arbitrary shape or form can be selected, for example from among granules, aggregates of particles, fibers, membranes and hollow fibers.

When a granular carrier is used in the manner of plasma separation, the average particle diameter of the carrier is preferably about 5 $\mu$m to 2,000 $\mu$m, more preferably about 20 to 1,000 $\mu$m, still more preferably about 30 $\mu$m to 800 $\mu$m. When it is used in the manner of direct hemoperfusion, average particle diameters below a lower limit of 80 $\mu$m sometimes make it difficult to perform direct hemoperfusion. Therefore, the average particle diameter is preferably not smaller than 80 $\mu$m and not larger than 2,000 $\mu$m and, for conducting direct hemoperfusion in a stable manner and from the adsorption capacity viewpoint, it is more preferably not smaller than 100 $\mu$m and not larger than 1,000 m$\mu$, particularly preferably not smaller than 120 $\mu$m and not larger than 800 $\mu$m.

For conducting stable direct hemoperfusion and for avoiding excessive blood cell activation more than necessary, it is desirable to restrict the average particle diameter distribution of particles. Excessively wide average particle diameter distributions induce blood cell adhesion and/or activation while narrow distributions tend to suppress the adhesion and/or activation caused by such physical factors as turbulent flows. In the practice of the invention, it is therefore preferable that at least 80% by volume of particles have diameters distributed within the range of the average particle diameter $\pm$ 75% and, for further inhibiting blood cell activation by the adsorbent itself and the adhesion resulting therefrom and for stably conducting direct hemoperfusion, more preferably within the range of $\pm$ 50%, particularly preferably within the range of $\pm$ 30%.

The average particle diameter, so referred to herein, is the value determined by taking a photograph of the granular carrier at an increased magnification using an optical microscope, for instance, measuring the particle diameters in the picture taken and dividing the sum of the diameters by the total number of the particles measured.

For the particle diameters measured, the standard deviation is calculated, and the average particle diameter distribution is shown in terms of mean $\pm$ standard deviation.

Usable as the means for measuring particle diameters in a picture taken are the measurement method using a scale photographed at the same magnification, the method comprising measuring particle diameters in the picture taken using vernier calipers or the like and then making corrections by means of the magnification, and the method comprising measuring the picture taken using image analyzing software (Image-Pro plus, product of Medical Cybernetics, Inc.), for instance. The number of particles to be measured is preferably 100 or more at a minimum.

The adsorbent of the invention can be produced, for example, in the following manner. One or more raw material monomer compounds are dispersed/suspended in a solvent having an appropriate viscosity (e.g. water). Suspension polymerization is carried out in the conventional manner while stirring the suspension to give the desired adsorbent.

Further, the adsorbent of the invention may also comprise activated carbon.

Usable as the activated carbon are, for example, fibrous activated carbon derived from phenolic fibers; coconut shell-derived activated carbon, petroleum pitch-derived activated carbon, peat-derived activated carbon, charcoal-based activated carbon and like granular activated carbon species.

The average particle diameter of the activated carbon is not particularly restricted but is preferably about 5 $\mu$m to 2,000 $\mu$m, more preferably about 20 to 1,000 $\mu$m, still more preferably about 30 to 800 $\mu$m. The average particle diameter can be measured in the above-mentioned method.

Among the adsorbent components each comprising a high-molecular compound or activated carbon, polystyrene, styrene-divinylbenzene copolymers and activated carbon are preferred and, in particular, styrene-divinylbenzene copolymers are preferred, from the lymphocyte proliferation rate viewpoint.

Now, the method of body fluid treatment according to the invention is described.

The method of body fluid treatment of the invention is a method for relieving the condition of inhibited lymphocyte proliferation by bringing a mammalian body fluid into contact with an adsorbent comprising the above-mentioned high-molecular compound having a water contact angle within the range of 40° to 98° in the presence of a divalent cation chelating agent in the manner of extracorporeal circulation or by bringing a mammalian body fluid into contact with the above-mentioned activated carbon-containing adsorbent in the manner of extracorporeal circulation.

In carrying out the extracorporeal circulation procedure according to the invention, the adsorbent mentioned above is preferably contained in a container. Such container will be described later herein.

The method of body fluid treatment for relieving the condition of inhibited lymphocyte proliferation specifically includes, but is not limited to, the following procedures.

(1) The procedure comprising filling a container having a body fluid inlet port and a body fluid outlet port, which is equipped with a filter allowing body fluid passage but preventing adsorbent passage, with the adsorbent, treating a body fluid of a patient in the manner of extracorporeal circulation, if necessary in the presence of a divalent cation chelating agent, using the adsorption device prepared in the above manner and then returning the thus-treated body fluid to the patient.

(2) The procedure comprising conducting body fluid treatment according to the above procedure (1), returning the body fluid treated to the patient and thereafter returning autologous lymphocytes activated in advance to the patient's body.

(3) The procedure comprising returning autologous lymphocytes activated in advance to the patient's body, performing body fluid treatment according to the above procedure (1) and returning the body fluid treated to the patient.

As the extracorporeal circulation procedure mentioned above under (1), there may be mentioned, for example, the procedure comprising extracorporeally circulating the body fluid by means of a body fluid transfer pump or the like for a certain period of contacting thereof with the adsorbent. Either of the following two techniques of such extracorporeal circulation procedure may be used: the plasma separation technique comprising separating plasma from blood cells using a plasma separation membrane, for instance, treating the plasma by means of the adsorption device mentioned above and returning the plasma, together with the blood cell fraction, to the patient, and the direct hemoperfusion technique comprising circulating blood directly without separating plasma from blood cells.

As for the contacting time, 15 minutes or a longer period of contacting is preferred and, from the adsorption performance viewpoint, about 30 minutes to 6 hours of contacting is more preferred. From the sufficient adsorption performance and cell treatment efficiency viewpoint, about 45 minutes to 4.5 hours of contacting is more preferred and about 60 minutes to 3 hours of contacting is still more preferred.

In carrying out the method of body fluid treatment of the invention using an adsorbent comprising a high-molecular compound having a water contact angle within the range of 40° to 98°, a divalent cation chelating agent or an anticoagulant having antithrombin activity can be used as an anticoagulant for the body fluid. These anticoagulants may also be used in combination. When a divalent cation chelating agent is used, the extracorporeal circulation method described above under (1) can be conducted by continuously introducing the divalent cation chelating agent into the patient's body fluid at the entrance thereof into the extracorporeal circulation circuit while adjusting the concentration of the agent in blood according to need. Thereafter, the blood is preferably passed directly through the adsorption device in the on-line manner or separated into plasma and blood cells using a commercially available plasma separation membrane or the like for treatment of the plasma in the adsorption device. In the case of passing the blood directly through the adsorption device in the on-line manner, a divalent cation chelating agent may be used singly as the anticoagulant but, for inhibiting the bradykinin formation or the like due to activation of the blood coagulation system, the combined use of a divalent cation chelating agent and an anticoagulant having antithrombin activity is more preferred.

When an active carbon-containing adsorbent is used as the adsorbent, it is preferred from the smooth fluid passage viewpoint that the body fluid be brought into contact with the adsorbent in the presence of an anticoagulant having antithrombin activity. In this case, a divalent cation chelating agent may be used in combination with the anticoagulant having antithrombin activity or a divalent cation chelating agent may be used singly.

As for the technique of introducing the anticoagulant having antithrombin activity into the body fluid, there may be mentioned, for example, the one-shot technique comprising injecting the anticoagulant into the tip of the flow of the patient's body fluid at the time of entrance of the body fluid into the extracorporeal circulation circuit in the extracorporeal circulation scheme, and the technique comprising injecting the anticoagulant beforehand into the patient prior to body fluid treatment, collecting a body fluid containing the anticoagulant from the patient and subjecting the same to the above-mentioned body fluid treatment.

The site of introduction of the divalent cation chelating agent and the anticoagulant having antithrombin activity is preferably located upstream of the adsorption device.

The divalent cation chelating agent to be used in the practice of the invention is not particularly restricted but includes, among others, EDTA (ethylenediaminetetraacetic acid) disodium salt, EDTA dipotassium salt, EDTA tripotassium salt, ammonium oxalate, potassium oxalate, citric acid, monosodium citrate, disodium citrate and trisodium citrate. Preferred among them are citrate salts; citrate solutions are more preferably used. The term "citrate solutions" as used herein means solutions containing at least one of citric acid, monosodium citrate, disodium citrate, trisodium citrate and the like. Further, divalent cation chelating agent solutions containing at least one of the above-mentioned ingredients are preferred, and commercially available divalent cation chelating agent solutions such as CPD, ACD-A, ACD-B and MAP solutions are more preferred.

The anticoagulant having antithrombin activity which is to be used in the practice of the invention is not particularly restricted but includes, among others, nafamostat mesylate, argatroban, heparin and low-molecular-weight heparin.

Heparin and low-molecular-weight heparin are preferred.

In the practice of the invention, the amount of the divalent cation chelating agent to be used is preferably such that the ratio (volume ratio) of the divalent cation chelating agent solution (e.g. such a citric acid solution as ACD-A or ACD-B solution) to the body fluid be 1:10 or more to 1:80 or less, more preferably 1:15 to 1:70, still more preferably 1:20 to 1:60. Higher levels of addition of the divalent cation chelating agent may possibly cause tetanic symptoms such as numbness of lips and/or limbs and vomiturition due to chelation of large amounts of ionized calcium in the blood. On the other hand, lower addition levels will allow platelets to adhere to the adsorbent, for instance, and such event may possibly cause a hindrance to blood passage.

When an anticoagulant having antithrombin activity is used in the practice of the invention, it is preferably used in an amount such that the concentration of the anticoagulant having antithrombin activity (e.g. heparin or low-molecular-weight heparin) in the body fluid amount to 0.01 IU/ml to 1.5 IU/ml, more preferably 0.05 IU/ml to 1.0 IU/ml, still more preferably 0.1 IU/ml to 1.0 IU/ml, particularly preferably 0.2 IU/ml to 1.0 IU/ml. Excessively high levels of addition of the anticoagulant having antithrombin activity will make it difficult to arrest bleeding, while excessively low levels of addition thereof will readily allow blood coagulation.

Since it is used at low concentration levels in blood, the anticoagulant having antithrombin activity may be continuously injected. Alternatively, it may be injected all at once in the one-shot manner so as to attain a predetermined concentration.

When injected all at once to attain a predetermined blood concentration, the divalent cation chelating agent may possibly cause shock symptoms and, therefore, it is preferably injected continuously so that the volume ratio may fall within the range mentioned above.

When autologous lymphocytes activated in advance are returned to the patient's body according to the procedure (2) mentioned above following the body fluid treatment mentioned above for the procedure (1), the activated lymphocytes are preferably returned to the body within a week following the completion of the extracorporeal circulation procedure. Since it is desirable that the return of lymphocytes be made before rising of the blood concentrations of lymphocyte proliferation inhibitors, the returning is preferably made within three days, more preferably within 1 day. Usable for lymphocyte activation are antibodies and the like in general use, for example an antibody against CD3 (OKT3) and that against CD28.

The procedure (3) is a method of relieving the condition of inhibited lymphocyte proliferation which comprises stimulating lymphocytes for activation thereof in advance with a drug or a like stimulator, or activating and proliferating autologous lymphocytes extracorporeally and returning autologous lymphocytes to the patient's body, and carrying out extracorporeal circulation using the adsorption device mentioned above. The period from lymphocyte stimulation for activation to extracorporeal circulation using the adsorption device is preferably not longer than 1 week, more preferably not longer than 3 days, still more preferably not longer than 1 day.

Although the method of body fluid treatment of the invention is not limited to these, the procedures (1) and (2) mentioned above are preferred as methods of relieving the condition of inhibited lymphocyte proliferation by extracorporeal circulation, and the procedure (2) is most preferred as a disease treatment method giving a high curative or therapeutic effect.

As mentioned hereinabove, an unknown mechanism or mechanisms may possible be involved in lymphocyte proliferation inhibition, and an unknown "lymphocyte proliferation inhibitor or inhibitors" may be involved in the mechanism or mechanisms. Therefore, the method of body fluid treatment of the invention can function as a method of adsorptively removing lymphocyte proliferation inhibitors.

Now, the system for extracorporeal circulation of the invention is described.

The system for extracorporeal circulation of the invention is either a system which comprises a body fluid transfer pump, an anticoagulant injection pump, and a device comprising a container wherein an adsorbent comprising the above-mentioned high-molecular compound having a water contact angle within the range of 40° to 98° is contained, or a system which comprises a body fluid transfer pump, and a device comprising a container wherein the above-mentioned active carbon-containing adsorbent is contained. This system can be used for relieving mammalian body fluids from the condition of inhibited lymphocyte proliferation in the manner of extracorporeal circulation and, more specifically, can be used in carrying out the above-mentioned method of body fluid treatment of the invention.

The shape, size and material of the container to be used in the adsorption device are not particularly restricted.

The shape may be an arbitrary one, for example a ball, box, bag, tube or column shape. As preferred specific examples, there may be mentioned transparent or semitransparent cylindrical containers with a capacity of about 0.1 to 400 ml and a diameter of about 0.1 to 10 cm.

The container can be made using an arbitrary structural material. As the structural material, there may specifically be mentioned unreactive polymers, biocompatible metals, alloys and glasses, among others.

As the unreactive polymers, there may be mentioned acrylonitrile-butadiene-styrene terpolymers and like acrylonitrile polymers; polytetrafluoroethylene, polychlorotrifluoroethylene, tetrafluoroethylene-hexafluoropropylene copolymers, poly (vinyl chloride) and like halogenated polymers; polyamides, polysulfones, polycarbonates, polyethylene, polypropylene, poly(vinyl chloride-acrylic) copolymers, polycarbonate-acrylonitrile-butadiene-styrene, polystyrene, polymethylpentene and so forth.

As the metallic materials useful as the container materials, there may be mentioned, among others, stainless steel, titanium, platinum, tantalum, gold and alloys of these as well as gold-plated alloy iron, platinum-plated alloy iron, cobalt-chromium alloys and titanium nitride-coated stainless steel.

Particularly preferred are materials resistant to sterilization or autoclaving; specifically, there may be mentioned silicone-coated glass, polypropylene, poly(vinyl chloride), polycarbonates, polysulfones, polymethylpentene, etc.

The container mentioned above has a body fluid inlet port and outlet port, and the outlet port is preferably equipped with a filter through which the body fluid and components contained therein can pass but the adsorbent cannot. The container is filled with the adsorbent.

The system for extracorporeal circulation of the invention, when the adsorbent used therein comprises a high-molecular compound having a water contact angle within the range of 40° to 98°, comprises, in addition to such adsorption device, a body fluid transfer pump for transferring the patient's body fluid to the adsorption device and an anticoagulant injection pump or pumps for injecting a divalent cation chelating agent, if necessary together with an anticoagulant having antithrombin activity, into the body fluid.

The system for extracorporeal circulation of the invention in which an active carbon-containing adsorbent is used as the adsorbent comprises, in addition to the adsorption device mentioned above, a body fluid transfer pump for transferring the patient's body fluid to the adsorption device. Preferably, it further comprises an anticoagulant injection pump or pumps for injecting a divalent cation chelating agent or/and an anticoagulant having antithrombin activity into the body fluid.

The site of injection of a divalent cation chelating agent and/or an anticoagulant having antithrombin activity is desirably located upstream of the adsorption device and, therefore, the anticoagulant injection pump is preferably disposed in connection with the adsorption device so that it may be found upstream of the device. The anticoagulant injection site is preferably nearer to the blood drawing port in the blood drawing line so that blood coagulation may be inhibited.

When a divalent cation chelating agent and an anticoagulant having antithrombin activity (e.g. heparin) are used in combination, the anticoagulant injection pump for injecting the divalent cation chelating agent and the anticoagulant injection pump for injecting the anticoagulant having antithrombin activity are preferably located near to each other; either one of them may be located upstream of the other.

In returning the blood to the patient, the blood may be passed through a warming apparatus for warming the blood beforehand so that the body temperature may be prevented from lowering.

When the system for extracorporeal circulation of the invention is to be used in the manner of plasma separation, it is preferred that the system further comprise a plasma separation membrane. In the plasma separation scheme, it is preferred that plasma and blood cells be separated from the blood and the plasma be passed through the adsorption device; therefore, the plasma separation membrane is preferably located in connection with the adsorption device at a site upstream thereof.

In the system for extracorporeal circulation of the invention, direct hemoperfusion is performed in the on-line manner, or plasma is separated from blood cells using a commercially available plasma separation membrane or the like and the plasma is treated in the body fluid treatment device; therefore, if the adsorbent becomes compacted, a sufficient body fluid flow will be no longer obtained, possibly resulting in prolongation of the time for treatment or in impossibility of continuing the treatment. Accordingly, the adsorbent is preferably one having a sufficient degree of mechanical strength (namely a rigid one) so that compaction may be prevented.

The term "rigid" is used herein to refer to a carrier less solvent-swellable and hardly deformable under pressure as compared with soft carriers such as dextran, agarose and acrylamide. A rigid carrier can be distinguished from a soft carrier in the following manner. Thus, a cylindrical column is uniformly packed with a carrier and an aqueous liquid is passed through the column. In the case of a rigid carrier, the relation between pressure loss and flow rate becomes almost linear while, in the case of a soft carrier, the carrier is deformed under pressures exceeding a certain point and the flow rate no longer increases as a result of compaction. A carrier showing the above linear relationship at least until 0.3 Kg/cm$^2$ is referred to herein as a rigid carrier.

The mammal, so referred to herein, includes humans and other mammals than humans, for example domestic animals such as cattle, horses, sheep and swine.

The body fluid so referred to herein includes blood and plasma. In addition, the body fluid includes other body fluids, such as ascetic fluid, lymph and intraarticular fluid and fractions derived from these as well as other living body-derived fluid components.

The term "lymphocytes" as used herein refers to T cells and B cells, among others, occurring in mammalian peripheral blood, lymph vessels and bone marrow. The lymphocytes also include cells which are neither T cells nor B cells, for example natural killer cells. The T cells are not particularly restricted but include helper T cells, cytotoxic T cells and killer T cells.

The term "poor lymphocyte proliferation" indicates that when patient's body fluid is added to a lymphocyte culture system, the lymphocyte proliferation rate is lower than the lymphocyte proliferation rate determined using a normal human-derived body fluid. The lymphocyte proliferation rate is the proliferation rate (number of cells after 7 days/ number of cells at the time of seeding) found after 1 week of lymphocytes cultivation at 37°C using a culture medium supplemented with 0.1% (v/v) to 20% (v/v) of a patient-derived body fluid treated with the adsorbent or a normal human-derived body fluid not yet treated with the adsorbent.

(Effects of the invention)

The present invention has made it possible to relieve the condition of inhibited lymphocyte proliferation and thus markedly increase the lymphocyte proliferation rate by adsorptively removing lymphocyte proliferation inhibiting factors, by extracorporeal circulation, from a body fluid derived from a subject with a disease (cancer, infectious disease, immunologic disease or like disease) against which a therapeutic effect is produced by extracorporeally activating lymphocytes and returning them into the body, for example a cancer subject with poor lymphocyte proliferation, without adsorbing factors necessary for lymphocyte proliferation from that body fluid. Further, the method of body fluid treatment has made it possible to carry out stable extracorporeal circulation treatment in the plasma separation mode or direct hemoperfusion mode, among others, while minimizing the loss of blood cells.

BEST MODE FOR CARRYING OUT THE INVENTION

The following examples specifically illustrate the present invention. These examples are, however, by no means limitative of the scope of the invention.
In the following examples etc., the water contact angle, molecular-weight exclusion limit and average particle diameter measurements were made in the following manner.

(1) Water contact angle

A flat and smooth film was prepared by compressing the high-molecular compound sample at a high pressure. The thus-obtained flat and smooth sheet specimen was placed horizontally, and a liquid drop was formed thereon using a microinjector. The size of the liquid drop was such that the contact diameter was about 1 to 2 mm. The contact angle was determined by measuring the angle formed upon allowing the liquid drop to advance toward the solid surface, at room temperature (20°C), using a reading microscope (having a magnification of about 20) equipped with a goniometer.

(2) Molecular-weight exclusion limit

Polystyrene beads differing in particle diameter were passed through a column packed with the adsorbent, and the pore size was determined on the basis of an excluded polystyrene beads-based exclusion curve. Then, the above-mentioned pore size was extrapolated to the spherical protein dextran the diameter and molecular weight of which were known, to thereby determine the molecular weight on the dextran equivalent basis; this was reported as the molecular-weight exclusion limit.

(3) Average particle diameter

The adsorbent in a wet state was developed on a dish and scores of particles were photographed for each field using a CCD camera so that the total number of particles will amount to be not less than 100 by counting. Then, the average particle diameter was calculated from the image captured using the particle diameter measurement software "Image-Pro plus" (product of Medical Cybernetics, Inc.).

(Example 1)

(1) Lymphocyte preparation

A winged needle for intravenous injection was stabbed into the brachial region of a normal subject and about 7.5 ml of blood was collected in a tube for lymphocyte separation (Vacutainer tube (product of Becton Dickinson and Company)). After blood collection, the Vacutainer tube was immediately subjected to 20 minutes of centrifugation at 3,000 rpm at room temperature. The lymphocyte layer was recovered and supplemented with 40 ml of physiological saline, and the resulting mixture was centrifuged at 1,500 rpm at 4°C for 5 minutes. This procedure was repeated several times for washing lymphocytes, and a lymphocyte suspension with a predetermined concentration was prepared by resuspending the lymphocytes in KBM 400 medium (product of Kohjin Bio Co., Ltd.).

(2) Preparation of a plate with OKT3 immobilized thereon

OKT3 (product of Dainippon Pharmaceutical) was diluted to a concentration of 5 $\mu$g/ml with physiological saline, and the dilution was distributed in 500-$\mu$l portions into the wells of a 24-well polystyrene microplate (product of Sumitomo Bakelite Co., Ltd.). After 2 hours of standing still at room temperature, the OKT3 solution was removed, and the plate was washed with two equal portions of physiological saline; a plate with OKT3 immobilized thereon was thus prepared.

(3) Preparation of an adsorbent

A monomer mixture composed of 100 parts by weight of divinylbenzene for industrial use (divinylbenzene content 57%), 100 parts by weight of toluene, 60 parts by weight of isoamyl alcohol and 1 part by weight of benzoyl peroxide (content 75%) was added to an aqueous solution composed of 572 parts by weight of water, 23 parts by weight of sodium chloride, 1 part by weight of polyvinyl alcohol and 0.03 part by weight of sodium nitrite, and the polymerization was carried out at 80°C in a nitrogen atmosphere for 5 hours with stirring so that droplets of the monomer mixture might be dispersed and suspended. The polymer particles formed were filtered off, washed with water and then deprived of such residual components as the solvent, monomer and initiator by extraction with acetone and again thoroughly washed with water and hot water. A granular porous material of styrene-divinylbenzene copolymers with an average particle diameter of about 400 $\mu$m was obtained (molecular-weight exclusion limit about 8 x $10^4$, water contact angle about 85°).

(4) Plasma treatment

The porous material was thoroughly washed with physiological saline and then 0.17 ml thereof was measured and placed in a cryotube. The physiological saline was thoroughly removed from the porous material, 1 ml of cancer patient's serum wherein ACD-A solution was at a volume ratio of 1:20 relative to plasma was added thereto, followed by 2 hours of incubation at 37°C with stirring (40 rpm) on a MIX rotor.

(5) Lymphocyte cultivation

The plasma treated in the above manner was added to the lymphocyte culture medium KBM 400 (product of Kohjin Bio Co., Ltd.) to a concentration of 9% (v/v). Using the resulting culture medium, a lymphocyte suspension with a lymphocyte number of 1.0 x $10^5$ cells/ml was prepared and sowed onto the previously prepared plate with OKT3 immobilized thereon in an amount of 444 $\mu$l/well (n = 3 wells). After 7 days of cultivation, lymphocytes were recovered and the number of cells was counted using a hemocytometer, and the lymphocyte proliferation rate was calculated according to the following formula (1). The results are shown in Table 1.

```
Proliferation rate (times) = number of lymphocytes after 7 days
  of cultivation/number of lymphocytes at the time of seeding
                                                          (1)
```

As a result, in the case of treatment of cancer patient's plasma with the adsorbent, the number of lymphocytes increased to 16.7 times (proliferation rate) the number of cells at the time of seeding.

(6) Blood passage experiment

The porous material obtained was washed with physiological saline supplemented with heparin (heparin sodium injection, Yoshitomi Pharmaceutical Industries, adjusted to a final heparin concentration of 1 U/ml) for equilibration with heparin. The porous material was then defoamed and thereafter 2.5 ml (sedimentation volume) thereof was packed into a minicolumn (made of polypropylene, inside diameter 13 mm, height 19 mm; product of Terumo Corp.). A poly (vinyl chloride) tube (inside diameter 1 mm, outside diameter 3 mm, length 70 cm) was connected to the column on the inlet side thereof, and a similar poly (vinyl chloride) tube (length 30 cm) was connected to the column on the outlet side thereof. Blood was carefully drawn from a normal subject using an 18G injection needle. The anticoagulants added thereto were ACD-A solution (solution having the composition: sodium citrate 2.20 g/dl, citric acid 0.80 g/dl and dextrose 2.20 g/dl) at a volume ratio of 1:20 relative to the blood and heparin at a final concentration of 0.5 IU/ml. A 30-ml portion of the blood supplemented with the anticoagulants was placed in an Erlenmeyer flask made of Teflon (registered trademark) (capacity 50 ml, Sanwa Co., Ltd.) and rotated at low speed by means of a stirrer chip in a constant-temperature bath maintained at 37°C, and the blood passage experiment was started at a flow rate of 2.9 ml/minute (linear velocity 2.6 cm/minute). Taking the point of time of the first appearance of the blood from the column outlet side as the starting point, the tip of the outlet side tube was returned to the blood pool and the perfusion experiment was carried out for 120 minutes. Blood samples for cytometry were taken at 30 minutes, 60 minutes and 120 minutes after the starting point.

(7) Cytometry

After the lapse of predetermined periods of time (30 minutes, 60 minutes and 120 minutes), blood samples were collected at the column inlet and on the outlet side, and blood cells (erythrocytes, leukocytes and platelets) in each sample were counted using a blood cell counter (Microcell Counter CC-180, Sysmex Corp.). Then, the percentage of cells that passed through the column was calculated for each of different cell species according to the formula (2) given below, and the results obtained are shown in Table 2.

```
Pass rate (%) = number of blood cells at column outlet/number
     of blood cells at column inlet x 100                (2)
```

The pass rates for erythrocytes and leukocytes were each about 100%. The pass rate for platelets was about 90%.

(Example 2)

Petroleum pitch-derived active carbon (DHP-1 (trade name), product of Kuraray Co., Ltd.) with an average particle diameter of about 500 $\mu$m was used as the adsorbent and the lymphocyte proliferation rate was determined in the same manner as in Example 1 . As a result, the number of lymphocyte increased to 10.6 times (proliferation rate) the number of cells at the time of seeding. The blood cell pass rates were evaluated in the same manner as in Example 1 except that heparin (final concentration 5 IU/ml) was used as the anticoagulant in lieu of ACD-A solution. The blood cell pass rates were about 100% for erythrocytes, about 80% for leukocytes, and about 60% for platelets.

(Example 3)

A styrene-divinylbenzene copolymer-based granular porous material with a molecular-weight exclusion limit of not higher than $1 \times 10^4$ and an average particle diameter of about 400 $\mu$m (water contact angle about 85°; prepared in the same manner as in Example 1 except that the amount of divinylbenzene for industrial use was changed to 115 parts by weight) was used as the adsorbent and the lymphocyte proliferation rate and blood cell pass rates were determined in the same manner as in Example 1. As a result, the number of lymphocytes increased to 11.5 times (proliferation rate) the number of cells at the time of seeding. The blood cell pass rates were about 100% for erythrocytes and leukocytes, and 80% to 85% for platelets.

(Example 4)

Adsorbent preparation:

A cellulose solution with a viscosity of about 1,000 cP was jetted, in the form of uniform droplets, into a gaseous phase under direct application of vibrations at a frequency of about 20,000 Hz to the solution. After causing the droplets to make a sufficient flight to take a spherical form, they were captured in a coagulation bath, deprived of the solvent and washed to give a porous cellulose material with an average particle diameter of about 400 $\mu$m (molecular-weight exclusion limit not higher than $3 \times 10^4$, water contact angle about 50°).

The above porous material was used as the adsorbent and the lymphocyte proliferation rate and blood cell pass rates were determined in the same manner as in Example 1. As a result, the number of lymphocyte increased to 6.9 times (proliferation rate) the number of cells at the time of seeding. The blood cell pass rates were about 100% for erythrocytes, about 80% for leukocytes, and about 80% to 90% for platelets.

(Example 5)

A porous cellulose material with a molecular-weight exclusion limit of not higher than $6 \times 10^4$ and an average particle diameter of about 400 $\mu$m (water contact angle about 40°; prepared by the method (vibration method) described in Japanese Kokai Publication Sho-63-117039, namely by causing a cellulose solution to form liquid droplets and capturing them in a coagulation bath for coagulation) was used as the adsorbent and the lymphocyte proliferation rate and blood cell pass rates were determined in the same manner as in Example 1. As a result, the number of lymphocyte increased to 5.7 times (proliferation rate) the number of cells at the time of seeding. The blood cell pass rates were about 100% for erythrocytes, about 70% to 80% for leukocytes, and about 85% to 95% for platelets.

(Comparative Example 1)

The lymphocyte proliferation rate was determined in the same manner as in Example 1 except that the patient's plasma was used without contacting with any adsorbent. In addition, the blood cell pass rates were determined in the same manner as in Example 1 except that an empty column not filled with any adsorbent was used. As a result, the number of lymphocytes increases to 3.9 times (proliferation rate) the number of cells at the time of seeding. The blood cell pass rates were about 100% for erythrocytes and leukocytes, and 95% to 100% for platelets.

(Comparative Example 2)

The lymphocyte proliferation rate and blood cell pass rates were determined in the same manner as in Example 1 except that the porous material used in Example 4 was used after binding dextran sulfate thereto via epichlorohydrin (molecular-weight exclusion limit not higher than $3 \times 10^4$, water contact angle about 35°). As a result, the number of lymphocytes increased to 3.6 times (proliferation rate) the number of cells at the time of seeding. The blood cell pass rates were about 100% for erythrocytes, about 80% to 95% for leukocytes, and about 80% to 90% for platelets.

(Comparative Example 3)

The lymphocyte proliferation rate and blood cell pass rates were determined in the same manner as in Example 1 except that the porous material used in Example 5 was used after binding dextran sulfate thereto via epichlorohydrin (molecular-weight exclusion limit not higher than $3 \times 10^4$, water contact angle about 30°). As a result, the number of lymphocytes increased to 3.2 times (proliferation rate) the number of cells at the time of seeding. The blood cell pass rates were about 100% for erythrocytes, about 80% to 90% for leukocytes, and about 80% to 85% for platelets.

The results of the lymphocyte proliferation rate evaluation in Examples 1 to 5 and Comparative Examples 1 to 3 are shown in Table 1, and the results of the blood cell pass rate evaluation are shown in Table 2. These results indicate that when a body fluid is brought into contact with a specific adsorbent in accordance with the invention, the lymphocyte proliferation rate can be markedly increased and, even in direct hemoperfusion, such blood cells as erythrocytes, leukocytes and platelets are scarcely lost and the adsorbent is high in blood passage performance.

Table 1

| Influence of use of patient's plasma on lymphocyte proliferation Lymphocyte proliferation rate after 7 days of cultivation (calculated according to equation 1)) | |
| --- | --- |
| | Lym phocyte proliferation rate (times) |
| Example 1 | 16.7±3.2 |
| Example 2 | 10.6±2.7 |
| Example 3 | 11.5±3.0 |
| Example 4 | 6.9±1.5 |
| Example 5 | 5.7±1.6 |
| Comp.Ex. 1 | 3.9±1.4 |
| Comp.Ex. 2 | 3.6±0.8 |
| Comp. Ex. 3 | 3.2±0.5 |
| | n=3±S.D |

Table 2

| Time course of the blood cell pass rate (unit: %) | | | | |
|---|---|---|---|---|
| | Blood Cell Species | 30 | 60 | 120 |
| Example 1 | Erythrocyte | 102±1 | 99±3 | 100±4 |
| | Leukocyte | 99±3 | 98±4 | 97±3 |
| | Platelet | 82±7 | 88±5 | 89±4 |
| Example 2 | Erythrocyte | 101±3 | 99±2 | 103±2 |
| | Leukocyte | 82±5 | 80±3 | 76±4 |
| | Platelet | 54±8 | 61±7 | 64±8 |
| Example 3 | Erythrocyte | 99±4 | 102±3 | 101±4 |
| | Leukocyte | 98±3 | 97±4 | 95±5 |
| | Platelet | 79±6 | 83±3 | 86±6 |
| Example 4 | Erythrocyte | 100±2 | 97±2 | 99±1 |
| | Leukocyte | 77±4 | 78±3 | 84±3 |
| | Platelet | 81±5 | 85±7 | 91±5 |
| Example 5 | Erythrocyte | 103±2 | 99±3 | 99±2 |
| | Leukocyte | 73±7 | 74±6 | 81±4 |
| | Platelet | 84±6 | 89±5 | 94±7 |
| Comparative Example 1 | Erythrocyte | 98±3 | 103±1 | 104±4 |
| | Leukocyte | 98±4 | 97±5 | 98±3 |
| | Platelet | 96±4 | 96±6 | 98±4 |
| Comparative Example 2 | Erythrocyte | 101±2 | 103±2 | 102±4 |
| | Leukocyte | 81±4 | 87±4 | 94±5 |
| | Platelet | 82±6 | 85±7 | 87±6 |
| Comparative Example 3 | Erythrocyte | 99±2 | 101±2 | 100±3 |
| | Leukocyte | 83±6 | 86±4 | 91±4 |
| | Platelet | 80±7 | 82±5 | 84±5 |
| | | | | n=3±S.D |

INDUSTRIAL APPLICABILITY

The present invention has made it possible to relieve the lymphocyte proliferation inhibition in lymphocyte culture and thus markedly increase the lymphocyte proliferation rate by adsorbing lymphocyte proliferation inhibiting factors from a body fluid derived from a subject with a disease against which a therapeutic effect is produced by extracorporeally activating lymphocytes and returning them into the body, for example a cancer subject with poor lymphocyte proliferation, without adsorbing factors necessary for lymphocyte proliferation from that body fluid. The invention is useful in that it provides an adsorbent for relieving the lymphocyte proliferation inhibition in lymphocyte culture using a subject's body fluid in activated autologous lymphocyte therapy, for instance, according to which a disease is treated or the progress of a disease is inhibited by taking immunocompetent cells (lymphocytes in particular) in blood out of the body, culturing them for stimulation/activation and for proliferation and again returning them into the body, as well as a method for proliferating lymphocytes which utilizes the above-mentioned adsorbent.

**Claims**

1. A method of body fluid treatment for relieving the condition of inhibited lymphocyte proliferation which comprises bringing a mammalian body fluid into contact with an adsorbent comprising a high-molecular compound having a water contact angle within the range of 40° to 98° in the presence of a divalent cation chelating agent in the manner of extracorporeal circulation.

2. The method according to Claim 1 wherein the divalent cation chelating agent is a citrate salt.

3. The method according to Claim 2 wherein a solution of the citrate salt as the divalent cation chelating agent is used at a volume ratio of 1:10 or more to 1:80 or less relative to the body fluid, and the body fluid contains heparin in an amount of 0.01 IU/ml to 1.5 IU/ml.

4. The method according to any one of Claims 1 to 3 wherein the molecular-weight exclusion limit of the absorbent is not higher than $1.5 \times 10^5$.

5. The method according to any one of Claims 1 to 4 wherein the high-molecular compound is an aromatic high-molecular compound.

6. The method according to Claim 5 wherein the aromatic high-molecular compound is a polystyrene or a styrene-divinylbenzene copolymer.

7. A method of body fluid treatment for relieving the condition of inhibited lymphocyte proliferation which comprises bringing a mammalian body fluid into contact with an active carbon-containing adsorbent in the manner of extracorporeal circulation.

8. The method according to any one of Claims 1 to 7 wherein the adsorbent is contained in a container.

9. A system for extracorporeal circulation which comprises a body fluid transfer pump, an anticoagulant infusion pump, and a device comprising a container wherein an adsorbent comprising a high-molecular compound having a water contact angle within the range of 40° to 98° is contained.

10. A system for extracorporeal circulation which comprises a body fluid transfer pump, and a device comprising a container wherein an active carbon-containing adsorbent is contained.

11. The system according to Claim 9 or 10 wherein the anticoagulant infusion pump is located upstream of the device.

12. The system according to any one of Claims 9 to 11 which further comprises a plasma separation membrane.

13. The system according to Claim 12 wherein the plasma separation membrane is located upstream of the device.

14. The system according to any one of Claims 9 to 13 for relieving the condition of inhibited lymphocyte proliferation in a mammalian body fluid.

## INTERNATIONAL SEARCH REPORT

| International application No. |
| --- |
| PCT/JP2006/306526 |

A. CLASSIFICATION OF SUBJECT MATTER
*A61M1/36*(2006.01), *B01J20/26*(2006.01)

According to International Patent Classification (IPC) or to both national classification and IPC

B. FIELDS SEARCHED

Minimum documentation searched (classification system followed by classification symbols)
A61M1/36, B01J20/26

Documentation searched other than minimum documentation to the extent that such documents are included in the fields searched
Jitsuyo Shinan Koho          1922-1996   Jitsuyo Shinan Toroku Koho   1996-2006
Kokai Jitsuyo Shinan Koho   1971-2006   Toroku Jitsuyo Shinan Koho   1994-2006

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | JP 2002-539178 A   (Japan Immuno Research Laboratories Co., Ltd.), 19 November, 2002 (19.11.02), Claims; Par. Nos. [0037], [0043], [0060], [0063], [0073]; Fig. 2 & US 6498007 B1          & WO 2000/055621 A2 | 9,11-14 |
| X | JP 56-125056 A   (Terumo Corp.), 01 October, 1981 (01.10.81), Page 2, upper right column, line 7 to lower right column, line 4; Fig. 2 (Family: none) | 10 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 14 June, 2006 (14.06.06) | 27 June, 2006 (27.06.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

## INTERNATIONAL SEARCH REPORT

| | International application No. |
|---|---|
| | PCT/JP2006/306526 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 1-8
   because they relate to subject matter not required to be searched by this Authority, namely:
   Claims 1 to 8 pertain to methods for treatment of the human body by therapy and thus relate to a subject matter which this International Searching Authority is not required, under the provisions of the PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the
☐ The additional search fees were accompanied by the applicant's protest and, where applicable, payment of a protest fee..

☐ The additional search fees were accompanied by the applicant's protest but the applicable protest fee was not paid within the time limit specified in the invitation.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP SHO56092824 A
- JP HEI08257398 A
- JP 2001218840 A
- JP 2003310751 A
- JP 2003339854 A
- JP 2004073618 A
- JP SHO63117039 A

### Non-patent literature cited in the description

- Kaimen to Koroido (Interface and Colloid. Shin-Jikken Kagaku Koza (Lectures in Experimental Chemistry, New Series. Maruzen Co., Ltd, 1977, vol. 18, 52